# EUROPEAN PATENT APPLICATION

(11) **EP 4 088 753 A1**
(43) Date of publication of application: **16.11.2022**
(21) Application number: 22173612.7
(22) Date of filing: 16.05.2022
(51) Int. Cl.: A61L 9/20, A61L 9/16, A61L 9/22

(54) **AN APPARATUS FOR PERFORMING AN AIR PURIFICATION AND SANITIZATION TREATMENT**

(30) Priority: 14.05.2021 IT 202100012560
(71) Applicant: Di Mauro, Concetto, 95045 Misterbianco (MT) (IT); Fichera, Fabrizio Fabian Julian, 95030 Tremestieri Etneo (CT) (IT)
(72) Inventor: Di Mauro, Concetto, 95045 Misterbianco (MT) (IT); Fichera, Fabrizio Fabian Julian, 95030 Tremestieri Etneo (CT) (IT)
(74) Representative: Simino, Massimo

(57) **Abstract**

An apparatus for performing an air and surface purification and sanitization treatment, comprising a body, inside which an air treatment path is defined between an inlet for the air to be treated and an outlet for the treated air, means being provided along said air path for performing an air purification and sanitization treatment, comprising a firs stage (I) for performing a passive filtration treatment and a second stage (II) with a photocatalytic cell having two facing plates (2) made of nanoporous ceramic and LED means (3, 4) which emit a UVC radiation to irradiate said one plate (2) with a UVC radiation.

## Description

The present invention relates to an apparatus for performing an air and surface purification and sanitization treatment as defined in the preamble of claim 1.

In various environments, such as domestic, industrial and hospital environments, public environments, i.e. indoor environments with a large number of people therein, such as offices, restaurants, waiting rooms, schools and others, as well as indoor environments such as a railway carriage, an aircraft or a ship, the need is strongly felt to be able to perform purification and sanitization treatments on the air and the surfaces, namely if there is little air change or if air change is provided by closed systems for ambient-air recycling.

In particular, there is the need to be able to purify air from particles/particulate and odors, thereby alleviating or even eliminating any problem associated with the presence or proliferation of bacteria, viruses, germs, molds, spores and allergens.

Thus, there is the need to treat the air of indoor environments with the purpose of making them healthier for people that remain therein or pass therethrough.

Various apparatuses are currently available for indoor air treatment, with a variety of technologies implemented therein, such as activated carbon filtration, UV-C irradiation-activated photocatalysis or cold plasma treatment, to obtain the desired results.

It shall be noted in this respect that the apparatuses are not suitable for universal use, in that such apparatuses comprise specific technologies designed to solve specific problems, such as purification or germicidal protection, and are not able to meet purification, sanitization and similar needs other than those specifically targeted.

It will be thus appreciated that an additional need to be met is the provision of an apparatus for performing air and surface purification and sanitization treatments that has a universal use, i.e. is suitable to purify and sanitize the treated air with different technologies to be used as needed to eliminate or reduce particles/particulate and odors, as well as to eliminate or alleviate problems caused by bacteria, germs, viruses, germs, molds, spores and allergens.

This invention is based on the problem of providing a apparatus for performing an air and surface purification and sanitization treatment that has such structural and functional characteristics as to fulfill the above needs, while obviating the above prior art drawbacks.

This problem is solved by an apparatus for performing an air and surface purification and sanitization treatment as defined in the features of claim 1.

In a further aspect, this problem is solved by a process of performing an air and surface purification and sanitization treatment as defined in claim 15.

Further features and advantages of the apparatus for performing an air and surface purification and sanitization treatment will be apparent from the following description of a few preferred embodiments thereof, which is given by way of illustration and without limitation with reference to the accompanying drawing, in which:

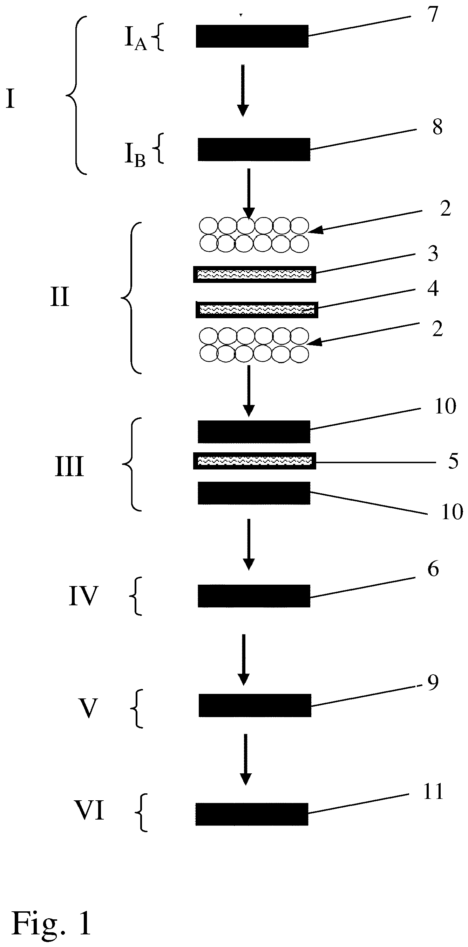

- Figure 1 is an exemplary schematic diagram of an air and surface purification and sanitization path through various stages of the apparatus for performing an air and surface purification and sanitization treatment according to the invention.

With reference to the accompanying figures, an apparatus for performing an air purification and sanitization treatment according to the invention comprises a body inside which an air treatment path is defined between an inlet for the air to be treated and an outlet for the treated air.

This air treatment path has various air treatment stages arranged in series therealong, and having means for performing air and surface purification and/or sanitization treatments, namely:
- a first stage I for performing a passive filtration treatment and
- a second stage II comprising:
   - a photocatalytic cell having at least one plate 2, preferably at least two parallel facing plates 2, made of microporous ceramic, for example with porosity values of less than 50 pm, more preferably with porosity values of less than 10 µm and even more preferably with porosity values of less than 10 µm and

   - LED means 3, 4 for emitting a UVC radiation to irradiate said at least one plate 2 with a UVC radiation, to thereby produce hydrogen peroxide.

According to a preferred embodiment, said at least one plate 2, or said at least two parallel facing plates 2 are made of nanoporous ceramic, for example with porosity values of less than 70 nm, more preferably less than 50 nm.

Preferably, the aforementioned LED means 3, 4 comprise:
- first LED means 3 for emitting UVC with a wavelength of 250 nm ±10 nm, preferably with a UVC radiation yield power ranging from 25 W to 30 W, more preferably with a UVC radiation yield power of about 28 W, which has shown to be very effective to act on viruses, bacteria and spores and ensure an effective disinfecting, purification and sanitizing action on air and surfaces, and
- second LED means 4, each strip having three LEDs, for emitting UVC with a wavelength of 270 nm ±5 nm, preferably a UVC radiation yield power ranging from 52 W to 60 W, more preferably with a UVC radiation yield power of about 58 W, which has shown to be very effective in disrupting and inactivating DNA and RNA of bacteria and viruses respectively.

Preferably, said at least one plate 2 is coated or sprayed with or comprises: titanium dioxide, silver, silver ions and/or apatite.

More preferably, said at least one plate 2 is coated or sprayed with or comprises: titanium dioxide, silver ions and apatite, to achieve a more effective photocatalysis due to the synergy caused by the concomitant presence of titanium dioxide, silver ions and apatite, which considerably increases hydrogen peroxide production.

It should be noted that the above LEDs 4 emitting UVC with a wavelength of 270 nm ±5 nm are also useful for photocatalysis activation.

According to a preferred embodiment, the aforementioned LED means comprise four distinct LED strips, each strip having single LED 3 for emitting UVC with a wavelength of 250 nm ±10 nm and two separate LEDs 4 for emitting UVC with a wavelength of 270 nm ±5 nm.

Preferably, the apparatus of the invention has a third stage III comprising:
- a titanium dioxide foam filter which is a bioactive nano-filter
- third LED means 5 for irradiating said titanium dioxide foam filter with UVC radiation.

Preferably, the aforementioned third LED means 5 emit UVC to irradiate said bioactive titanium dioxide foam nano-filter with a wavelength of 270 nm ±5 nm which has shown to be very effective in this stage III in causing further photocatalysis to occur besides a mechanical filtration performed by the aforementioned bioactive titanium dioxide foam nano-filter, resulting in the production of hydrogen peroxide.

For this purpose, according to a preferred embodiment, the aforementioned third LED means 5 comprise an LED strip with three LEDs onboard for irradiating the titanium dioxide surface of the filter with a UVC radiation having a wavelength of 270 nm ±5 nm and a UVC radiation yield power ranging from 19 W to 26 W, more preferably a UVC radiation yield power of about 22 W.

Preferably, the aforementioned first stage I for performing a passive filtration treatment comprises a stage I_{A} with an activated carbon filter 7, more preferably an activated carbon filter 7 comprising silver ions and potassium permanganate. It should be noted that potassium permanganate allows ozone to decay from O₃ to O₂.

Preferably, the aforementioned first stage I for performing a passive filtration treatment comprises a stage I_{B} with a high efficiency ULPA - Ultra Low Penetration Air - filter 8.

Preferably, said bioactive titanium dioxide foam nano-filter of the third stage III comprises two parallel and facing plates 10, made of titanium dioxide foam irradiated by said third LED means 5.

Preferably, the apparatus of the invention has a fourth ionization stage IV comprising an ionizer 6 suitable to release from 200×10⁶ to 500×10⁶ negative ions 3 per cm³.

Preferably, the apparatus of the invention has a fifth stage V with means 9 for performing a cold plasma treatment on the air passing through said fifth stage and preferably said fifth stage V comprises means 9 for performing a cold plasma treatment on the air passing through said fifth stage V.

Preferably, the aforementioned means 9 for performing a cold plasma treatment comprise two distinct, spaced apart cold plasma producing devices, to increase ion concentration in the environment, preferably to saturation.

Preferably, the apparatus of the invention has a sixth stage VI comprising an ozonizer 11 for performing an ozonization treatment on the air passing through said sixth stage VI, preferably an ozonizer 11 capable of producing from 5 g/h to 100 g/h of ozone.

Preferably, multicolor light means, preferably a multicolor LED strip, are provided at an outer portion of the body of the apparatus of the invention, for performing a chromotherapy treatment in an environment in which said apparatus is positioned.

Furthermore, the apparatus of the invention comprises motor means, ventilation means, actuation means and control means required to ensure proper operation of said apparatus, while also ensuring an air flow along said air path from said inlet for the air to be treated to said outlet for the treated air.

Preferably, the apparatus of the invention comprises:
- temperature detectors;
- humidity detectors;
- pm 1.0, 2.5, 10.0 and 0.1 detectors and
- gas detectors
for detecting temperature values, humidity values, the presence of particles/particulate and the presence of gases in the air to be treated with said apparatus and transmitting the detected values to control means.

Preferably, the apparatus of the invention comprises communication and interfacing means for providing data to a remote Artificial Intelligence server and obtaining a specific operating mode to be used according to the specific type of data that has been detected and transmitted.

According to the invention, the process for performing an air and surface purification and sanitization treatment by means of the apparatus of the invention as described above comprises the steps of:
- drawing air to be treated into the body of said apparatus through the aforementioned inlet;
- performing a first passive mechanical filtration treatment on the air that has been drawn within the aforementioned first stage I to purify all or part of the air drawn through said inlet from odors and/or particles and
- subjecting the drawn air to a photocatalytic treatment within the aforementioned second stage II, thereby causing a germicidal, virucidal and bactericidal effect in the treated air.

Preferably, the process of the invention comprises a step of subjecting the treated air to a filtration step that is carried out inside said third stage III by means of said bioactive titanium dioxide foam nano-filter irradiated with UVC.

Preferably, the process of the invention comprises a step of subjecting the treated air to ionization within said fourth ionization stage IV.

Preferably, the process of the invention comprises a cold plasma air treatment step in said fifth stage V.

Preferably, the process of the invention comprises an ozonization step on the air passing through said sixth stage VI.

Preferably, the above step of performing a first passive mechanical filtration treatment on the drawn air is carried out in a stage I_{A} with an activated carbon filter 7 comprising silver ions and potassium permanganate.

Preferably, the above step of performing a first passive mechanical filtration treatment on the drawn air is carried out in a stage I_{B} by means of a high efficiency ULPA - Ultra Low penetration Air - filter 8.

Preferably, the air that has been drawn by said inlet enters the first stage I and then sequentially flows through said second stage II, said third stage III, said fourth stage IV, said fifth stage V and, finally, said sixth stage VI, before exiting the body of the apparatus through the aforementioned outlet for the treated air.

Concerning the sixth stage VI, it should be noted that the ozonizer therein may also be selectively and independently activated, irrespective of the means and technology of the remaining stages of the apparatus.

Preferably, the process of the invention comprises the steps of:
- detecting operating parameters to be used and/or parameters detected by these sensors and transmitting them to said remote Artificial Intelligence server and
- receiving, from said remote Artificial Intelligence server, operating modes to be used to treat the air to be purified and sanitized according to the conditions and specific data detected and transmitted to said remote Artificial Intelligence server.

Instead of or in combination with the above, the apparatus of the invention may be actuated by a user manually, possibly also using a remote control or remotely by means of a suitable transmission protocol, e.g. WIFI.

It will be appreciated from the above that the apparatus for performing air and surface purification and sanitization of the present invention fulfills the above mentioned need and also obviates prior art drawbacks as set out in the introduction of this disclosure. This is because the presence of a microporous or nanoporous ceramic plate irradiated with the UVC radiation emitted by LED means, in the aforementioned first stage I, considerably increases the effectiveness of air treatment. In particular, viruses, bacteria, germs, molds, spores and allergens.

A further advantage of the apparatus for performing an air and surface purification and sanitization treatment according to the present invention is the possibility of using a single apparatus to perform purification and de-odorization treatments or treatments aimed at eliminating or alleviating the effects caused by any bacteria, viruses, germs or spores in the air and in the surfaces to be treated, with the monitoring and control means of the apparatus actuating the relevant stages from time to time and for the required duration.

One more advantage of the apparatus for performing an air and surface purification and sanitization treatment according to the present invention is its ability to act in each environment in which it is placed immediately after being positioned therein and connected to the mains.

Those skilled in the art will obviously appreciate that a number of changes and variants may be made to the apparatus as described hereinbefore to meet specific needs, without departure from the scope of the invention, as defined in the following claims.

## Claims

1. **An apparatus for performing an air and surface purification and sanitization treatment,** comprising a body, inside which an air treatment path is defined between an inlet for the air to be treated and an outlet for the treated air, means being provided along said air path for performing an air purification and sanitization treatment, comprising:
- a first stage (I) for performing a passive filtration treatment and
- a second stage (II) with a photocatalytic cell, **characterized in that** said photocatalytic cell of said second stage (II) comprises:
- at least one plate (2), preferably two facing plates, made of microporous ceramic and
- LED means (3, 4) which emit a UVC radiation to irradiate said at least one plate (2) with a UVC radiation.

2. An apparatus as claimed in claim 1, wherein said at least one plate (2) is coated and sprayed with or comprises: titanium dioxide, silver, silver ions and/or apatite.

3. An apparatus as claimed in claim 1 or 2, wherein said LED means (3, 4) comprise:
- first LED means (3) for emitting UVC with a wavelength of 250 nm ±10 nm and/or second LED means (4) for emitting UVC with a wavelength of 270 nm ±5 nm.

4. An apparatus as claimed in any of claims 1 to 3, comprising a third stage (III) comprising:
- a titanium dioxide foam filter which is a bioactive nano-filter
- third LED means (5) for irradiating said titanium dioxide filter with a UVC radiation, preferably a UVC radiation with a wavelength of 270 nm ±5 nm and wherein, preferably, said third stage (III) comprises two parallel plates (10) made of titanium dioxide foam, facing each other and irradiated by said third LED means (5).

5. An apparatus as claimed in any of claims 1 to 4, comprising a fourth ionization stage (IV), preferably said fourth ionization stage (IV) comprising an ionizer (6) suitable to release from 200×10⁶ to 500×10⁶ negative ions per cm³.

6. An apparatus as claimed in any of claims 1 to 5, comprising a fifth stage (V) with means (9) for performing a cold plasma treatment on the air passing through said fifth stage, said means (9) for performing a cold plasma treatment preferably being two distinct and separate groups.

7. An apparatus as claimed in any of claims 1 to 6, comprising a sixth stage (VI) comprising an ozonizer for performing an ozonization treatment on the air passing through said sixth stage (VI), preferably an ozonizer (11) capable of producing from 5 g/h to 100 g/h of ozone.

8. An apparatus as claimed in any of claims 1 to 7, wherein said first stage (I) for performing a passive filtration treatment comprises a stage (I_{A}) with an activated carbon filter (7) and/or a stage (I_{B}) With a high efficiency ULPA - Ultra Low penetration Air - filter (8), wherein, preferably, said activated carbon filter (7) comprises silver ions and potassium permanganate.

9. An apparatus as claimed in any of claims 1 to 8, wherein said at least one plate (2) of said second stage (II) is made of nanoporous ceramic.

10. An apparatus as claimed in any of claims 1 to 9, comprising:
- temperature detectors;
- humidity detectors;
- pm 1.0, 2.5, 10.0 and 0.1 detectors and
- gas detectors
which are suitable for detecting temperature, humidity, particulate matter and gas values and transmitting them to control means.

11. An apparatus as claimed in any of claims 1 to 10, comprising communication and interfacing means for providing data to a remote Artificial Intelligence server and obtaining a specific operating mode to be used according to the specific type of data that has been detected and transmitted.

12. **A method of performing an air and surface purification and sanitization treatment** by an apparatus as claimed in any of claims 1 to 11, said method comprising the steps of:
- drawing air to be treated into said body of said apparatus through said inlet;
- performing a first passive mechanical filtration treatment on the air that has been drawn into said first stage (I) to purify all or part of the air drawn through said inlet from odors and/or particles and
- subjecting the drawn air to a photocatalytic treatment in said second stage (II) thereby causing a germicidal, virucidal and bactericidal effect in the treated air.

13. A method as claimed in claim 12, comprising a step of:
- subjecting the treated air to a filtration step with an UVC-irradiated titanium dioxide foam filter inside said third stage (III);
- subjecting the treated air to ionization within said fourth ionization stage (IV);
- performing a cold plasma air treatment step in said fifth stage (V) and/or
- performing an ozonization step on the air passing through said sixth stage (VI).

14. A process as claimed in claim 12 or 13, wherein said step of performing a first passive mechanical filtration treatment on the drawn air is carried out:
- in a stage (I_{A}) with an activated carbon filter (7) with silver ions and potassium permanganate and/or
- using a high efficiency ULPA - Ultra Low penetration Air - filter (8).

15. A method as claimed in any of claims 12 to 14, wherein said apparatus comprises means for detecting temperature, humidity, particulate and/or gas values and communication and interfacing means for exchanging data with a remote Artificial Intelligence server, said method comprising the steps of:
- detecting and transmitting operating parameters and/or parameters detected by said sensors to said remote Artificial Intelligence server and
- receiving, from said remote Artificial Intelligence server, operating modes to be used to treat the air and the surfaces to be filtered and sanitized according to the conditions and specific data detected and transmitted to said remote Artificial Intelligence server.
